# EUROPEAN PATENT SPECIFICATION

(11) **EP 1 711 818 B1**
(45) Date of publication and mention of the grant of the patent: **26.09.2012**
(21) Application number: 04809060.9
(22) Date of filing: 16.12.2004
(51) Int. Cl.: G01N 33/53, G01N 33/573, G01N 33/68, G01N 33/569

(54) **A TEST KIT FOR DETECTING PERIODONTAL DISEASE**
TESTKIT ZUM NACHWEIS EINER PERIODONTALEN ERKRANKUNG
KIT DE TEST POUR DETECTER UNE MALADIE PARODONTALE

(30) Priority: 30.01.2004 US 540296 P; 30.01.2004 SE 0400191
(43) Date of publication of application: 18.10.2006
(60) Divisional application: 12173024.6
(73) Proprietor: Tendera AB, 411 26 Göteborg (SE)
(72) Inventor: BENGTSSON, Thomas, S-416 54 Göteborg (SE); WIKSTRÖM, Maude, S-411 33 Göteborg (SE)
(74) Representative: Zakrisson, Ulrika
(86) International application number: PCT/SE2004/001883
(87) International publication number: WO 2005/073721

(56) References cited:
- US-A- 5 328 829
- US-A1- 2002 012 944
- US-B1- 6 280 687
- CHAPPLE I.L.C.: 'Periodontal disease diagnosis: current status and future developments' JOURNAL OF DENTISTRY vol. 25, no. 1, 1997, pages 3 - 15, XP002986131
- COX S.W. ET AL: 'A simple, combined fluorogenic and chromogenic method for the assay of proteases in gingival crevicular fluid' JOURNAL OF PERIODONTAL RESEARCH vol. 25, 1990, pages 164 - 171, XP000407114
- TAN K.S. ET AL: 'Bacteroides forsythus prtH genotype in periodontitis patients: occurrence and association with periodontal disease' JOURNAL OF PERIODONTAL RESEARCH vol. 36, 2001, pages 398 - 403, XP002986132
- ELEY B.M ET AL: 'Correlation Between Gingivain/Gingipain and Bacterial Dipeptidyl Peptidase Activity in Gingival Crevicular Fluid and Periodontal Attachment Loss in Chronic Periodontitis Patients. A 2-Year Longitudinal Study' JOURNAL PERIODONTAL vol. 67, 1996, pages 703 - 716, XP002986133
- ARMITAGE G.C. ET AL: 'Longitudinal Evaluation of Elastase as a Marker for the Progression of Periodontitis' JOURNAL PERIODONTAL vol. 65, 1994, pages 120 - 128, XP002986134
- LEE EN ET AL: "Potential role of vascular endothelial growth factor, interleukin-8 and monocyte chemoattractant protein-1 in periodontal diseases.", KAOHSIUNG JOURNAL OF MEDICAL SCIENCES, vol. 19, no. 8, August 2003 (2003-08), pages 406-415, XP008082383,
- OKADA H ET AL: "Diagnostic strategies of periodontitis based on the molecular mechanisms of periodontal tissue destruction.", ORAL DISEASES, vol. 2, no. 1, March 1996 (1996-03), pages 87-95, XP008082386,
- JIN L J: "Relationship of changes in interleukin-8 levels and granulocyte elastase activity in gingival crevicular fluid to subgingival periodontopathogens following non-surgical periodontal therapy in subjects with chronic periodontitis.", JOURNAL OF CLINICAL PERIODONTOLOGY, vol. 29, no. 7, July 2002 (2002-07), pages 604-614, Denmark
- NAKAGAWA T ET AL: "Functional characteristics of antibodies induced by Arg-gingipain (HRgpA) and Lys-gingipain (Kgp) from Porphyromonas gingivalis", ORAL MICROBIOLOGY AND IMMUNOLOGY, vol. 16, no. 4, August 2001 (2001-08), pages 202-211,
- HIROSE K ET AL: "Levels of Porphyromonas gingivalis Fimbriae and inflammatory cytokines in gingival crevicular fluid from adult human subjects.", MICROBIOLOGY AND IMMUNOLOGY, vol. 41, no. 1, 1997, pages 21-26,

## Description

### Technical field

The present invention relates to a test kit for detecting periodontal disease. The present disclosure further relates to a method for the diagnosis and prediction of risk for progress of periodontal disease.

### Technical background

Periodontitis affects approximately 7-15% of the adults in the western world, making it one of our mcst common diseases. It is a multifactor disease where the presence of pathogenic bacteria in a pocket between tooth and gum is a necessary but not sufficient criterion. The host inflammatory and immune system also play a crucial role in the development of the disease. The progression of periodontitis is thought to be a chronic inflammatory response to subgingival bacteria, resulting in a destruction of the tooth's supportive tissues. It is cyclic in its behaviour and may remain unnoticed in its early phase.

The destructive process is thought to be the result of a complex interaction between the defence system of the host and specific bacterial species in the periodontal pocket. Pathogenic bacteria involved in the appearance and progression of periodontal disease include, but are not limited to, *Porphyromonas gingivalis* (formerly *Bacteroides gingivalis), Bacteroides forsythus* (now also called *Tannerella forsythensis), Actinobacillus actinomycetemcomitans, Triponema denticola* and *Prevotella intermedia .*

In later years the importance of the bacteria's virulence products (mainly toxins and enzymes) has been widely studied and is believed to play a major role in the pathogenesis (Eley and Cox (2003)). Bacteria are thought to go through different growth phases and during these phases to be more or less destructive in the periodontal pocket. Active bacteria produce virulence products to help its survival and nutrition in the periodontal pocket. During periods of elevated bacterial activity those virulence products contribute to the destruction of the tooth's supportive tissues and the reduction in effectiveness of the hosts' defence systems.

Today dentists assess periodontitis by measuring the probing depth of the periodontal pocket, examining x-ray images of the tooth attachment to the alveolar bone and refer to bleeding on probing. Risk factors include smoking habits, stress and a family history of periodontitis. The method relies heavily on the subjective expertise of the dentist. Probing depth is only a measurement of the historical attachment loss, thus giving little help in the actual occurrence of Periodontitis or the future progression thereof. Bleeding on probing could indicate a healing process instead of a destructive one.

Occasionally a microbiological sample is taken and sent of to a lab for analysis either by cultivation or DNA-techniques (Checkerboard DNA-DNA hybridization technique developed by Socransky (1994)). However, the answer is obtained in about a week's time and only shows the presence of certain bacteria, which not necessarily indicates periodontitis.

Vast amounts of work has been done to find a chemical compound, mainly a protein, such as an an enzyme or a cytokine, in fluids from the oral cavity of a patient, such as gingival crevicular fluid (GCF), that can diagnose or predict the progression of periodontitis.

Up until now there have been a number of tests and assays developed (Armitage (2003)). These assays have been dedicated to identifying bacteria, bacterial virulence products or host proteins.

Host derived proteins that have been investigated for their diagnostic or prognostic value in periodontitis include mainly products from the human inflammatory system. The role of these proteins is to orchestrate the inflammatory and immune response, remodelling of the tissue or to help in the killing of the invading bacteria.

The most studied host derived proteins intended for diagnosing periodontitis include the natural serine proteases (cathepsin G, azurocidin, proteinase 3, elastase), collagenase, aminotransferases (US Patents nos 4,981,787, 5,834,226 and 4,801,535), alkaline phosphatase, β-glucuronidase (US Patent no 6,277,587), dipeptidyl pedidase, neutrophil gelatinase-related lipocalin (US Patent no 5,866,432), matrix metalloproteinases (US Patents nos 5,736,341 and 6,280,687) and cytokines such as interleukins (especially IL-1β (US Patent no 5,328,829), IL-6 and IL-8) and inflammatory mediators such as prostaglandin E₂ and tumour necrosis factor-α (TNF-α).

Matrix metalloproteins (MMP's) have been suggested as a marker for periodontal disease. US Patent no 5,736,341 discloses detection of the presence of MMP-8, US patent no 5,866,432 discloses detection of the presence of neutrophil gelatinase-related lipocalin and US patent no 6,280,687 discloses the presence of MMP-13. Theses three patents suggest a rapid chair side test based on the immunochromatographic principle for the diagnosis and progression prediction of periodontitis. However, in the information paper from the American Association of Periodontology by Oringer (2002) it is argued that additional studies are needed in order to verify the roll of MMP's in the progression of periodontal disease.

US Patent no 6,406,873 claim that two inflammatory mediators (plasminogen activator inhibitor 2 and tissue plasminogen activator) alone or in combination can diagnose periodontitis.

US Patent no 5,248,595 describes a method to simultaneously analyse up to three different periodontal pathogens.

Chapple (1997) has reviewed the traditional and currently employed methods of periodontal diagnosis and concludes that detection of markers, such as the presence of alkaline phosphatase in gingival crevice fluid, are more sensitive and specific as compared to clinical assessments, such as analysing tissue colour, probing pocket depth and measuring tooth mobility. Chapple also concludes that combining two or more such markers may produce the most accurate means for diagnosing ongoing or future disease activity, but shows no such combinations.

Jin et al. (1999) have been investigating the correlation between presence of periodontal pathogens i.e. bacteria, by using DNA probing methods, in the periodontal pocket and elastase in the gingival crevice fluid (GCF) .

Nisengard et al. (1992) have described a rapid latex agglutination test for the presences of *P. gingivalis*, *A*. *actinomycetemcomitans* and P. *Intermedia.*

Lamster et al (1994) have investigated clinical attachment loss and it's correlation to β-glucuronidase from human leukocytes.

Eley and Cox (1996) have developed a chair-side test based on enzyme substrate specific to the gingipain from *P. gingivalis.*

US 2002/0012944 (Xiao et al) discloses that the existence and the extent of periodontal disease can be diagnosed by measuring plasminogen activator inhibitor 2 (PAI-2) and/or tissue plasminogen activator (t-PA) levels in gingival crevicular fluid (GCF). Levels of PAI-2 and t-PA in GCF rise sharply in context of periodontal disease, and they also correlate with the severity of disease at different sites in the same patient.

Okada, H et al (Oral Diseases (1996) 2, 87-95) discloses diagostic strategies based on the molecular mechanisms causative of periodontitis. Bacterial factors such as bacterial cells and proinflammatory cytokines or mediators were studied. The authors state that diagnostic markers should be pathogenic and inflammatory factors participating in periodontal connective tissue and bone destruction.

En Lee et al (Kaohsiung J Med Sci (2003) 19, 406-414) presents a study investigating the activation of inflammatory processes in periodontal disease by quantitative assessment of VEGF, MCP-1 and IL-8 in GCF and of bacteria in subgingival plaque samples. It was found that there was significantly more VEGF and IL-8 in chronic periodontitis and gingivitis sites than in periodontally healthy sites.

Up to date, many methods for assessing periodontal disease activity have been developed. However, none of these above mentioned methods provide an specific and sensitive enough assay to diagnose the periodontitis and the destructive pattern thereof. One consequence of unspecific methods is that several patients will be treated without actually having periodontitis. Clinical observations such as probing depth are not reliable enough because deep pockets do not necessarily harbour an ongoing inflammation, radiographic evaluations have to be combined with detailed clinical observations in order to give an accurate diagnosis and the mere presences of pathogenic bacteria in the periodontal pocket do not ac-curately reflect disease activity. Moreover the so far developed diagnoses based on enzymatic methods for host or bacteria derived proteins have not been sufficiently specific due to the fact that an enzyme substrate can be cleaved by a multitude of different enzymes.

Different cytokines have also been studied but no rapid and specific tests have been designed.

Thus the dentist is in need of a chair side test kit that preferably:
- is rapid, yielding results within a few minutes.
- requires a minimum amount of time and work effort.
- is robust and can be treated rough in the clinic's environment.
- provides easily interpreted results.
- have a long shelf-life in room temperature or in a fridge.
- fits the tray at the dental clinic, is environmentally friendly and provides good patient information material.

### Summary of the invention

One object for the present invention is to overcome the drawbacks of the prior art and to provide a test kit that meets the present needs of the dentists. More specifically, the present invention aims to provide a test kit for detecting periodontal diseases that is specific, sensitive and easy to use.

The inventors of the present invention have found that a test kit allowing the detection of the co-existence of a substance originating from bacteria and a substance originating from the human immune of inflammatory system can be used for this purpose.

The present invention can be used to help dentists and dental hygienists to provide their patients with a more efficient treatment of periodontal disease. The test kit and the method according to the invention may be used for screening suspected teeth, to follow up previous treatment, to decide the most appropriate form of treatment, to choose the right kind of antibiotics and to communicate to the patient the importance of good everyday dental hygiene.

In a first aspect, the present invention relates to a test kit for diagnosing periodontal disease in a patient by analysing a sample from the oral cavity of the patient, said kit comprising a first detection assay for detecting a first substance originating from bacteria, said first detection assay comprising:
i. a first antibody exhibiting selective binding to said first substance, and
ii. means for generating a detectable response,
said first substance being arg-gingipain from *Porphyromonas gingivalis,* and a second detection assay from detection of a second substance originating from the immune or inflammatory system of the patient, said second detection assay comprising:
i. a second antibody exhibiting selective binding to said second substance, and
ii. means for generating a detectable response,
said second substance being a human neutrophil elastase.

Preferably, said patient is a mammal, most preferably a human.

The result from said first detection assay in combination with the result from said second detection assay is used to detect periodontal disease.

The second substance is a human neutrophil elastase.

The co-existence of both a first substance of bacterial origin and a second substance as defined above, human neutrophil elastase, should indicate both active bacteria and an active immune or inflammatory system, and this indicates periodontal destruction.

It is clear that even though it is well established in the scientific community that periodontal disease is a multi-factorial disease no one has yet developed a test to analyse both bacterial and host derived products in a sample.

The test kit may also comprise a third detection assay comprising at least a third affinty ligand having a binding site for binding of a third substance originating from bacteria or from the immune or inflammatory system of the patient as defined above, preferably from bacteria, and may in some embodiments even comprise further detection assays for binding of additional substances.

The use of antibodies is advantageous over other methods (e.g. enzymatic methods) since antibodies, once developed and tested for cross-reactivity, are highly specific for their target and are able to detect chemical substances other than enzymes, e.g. toxins and cytokines. Furthermore, methods for development of new antibodies against new antigens are well known to those skilled in the art.

Preferably said detection assays in the test kit according to the present invention comprises immunochromatographic assays.

Among the advantages of using immunochromatographic assays/methods is that they are easily produced and used, have a long shelf-life, yields a quick answer and can be designed to be very specific for the substances intended to be detected.

Said test kit further preferably comprises a support provided with a sample reservoir for receiving a sample, wherein said first and second detection assays are arranged on said support in contact with said sample reservoir, directly or via a removably arranged separating means which separates said sample reservoir from said detection assays. Said kit may also comprise additional buffers, preferably in a buffer reservoir separate from said sample reservoir, for dilution and adaptation of said sample for said detection assays, and at least one sampling device for obtaining the sample.

The individual detection assays comprised in the test kit according to the present invention are provided together. In the case where the detection assays are sold separately, samples of GCF etc taken concomitantly are analysed separately on each assay, and the results are combined for the diagnosis of periodontal disease.

The test kit according to the present invention provides for a chair-side test for periodontal disease, wherein the dentist or a dental hygienist takes e.g. a GCF-sample from a periodontal pocket. The sample is analysed by the assays provided in the test kit and the results from these assays is judged according to predefined criteria to evaluate the occurrence of ongoing periodontal disease.

A method for diagnosing periodontal diseases and/or predicting the risk for progress of said diseases comprises analyzing a sample from the oral cavity of a patient for the presence of at least a first substance originating from bacteria and the presence of a second substance originating from the immune system or inflammatory system of the patient, the first and second substances beeing as defined above.

Furthermore, said diagnostic method may also comprise a method to detect the presence of additional substances as above.

More preferably at least one of said first and second methods comprises using an immunochromatographic assay.

### Brief description of the drawing

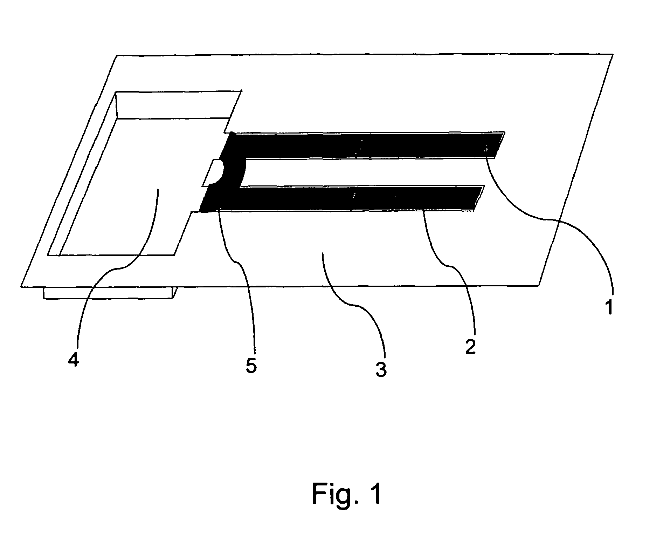

Figure 1 shows an embodiment of a test kit wherein two immunochromatographic assays are arranged on a support equipped with a sample reservoir.

### Detailed description of the invention

The present invention relates to a test kit for detecting periodontal disease in a patient by analysing a sample from the oral cavity of the patient, wherein said kit at least comprises a first detection assay comprising at least a first affinity ligand having a binding site for binding of a first substance originating from bacteria, and a second detection assay comprising at least a second affinity ligand having binding site for binding a second substance originating from the immune or inflammatory system of the patient according to claims 1-6.

Preferably, said sample from the oral cavity of a patient is gingival crevicular fluid, peri-implant sulcus fluid, saliva or a mouth rinse sample.

Gingival crevicular fluid (GCF) is a fluid that flows from the periodontal pocket into the oral cavity. In case of inflammation, GCF contains inflammatory cells, bacteria and their by-products respectively and it contents may be used as a marker for destructive periodontitis. Collecting the GCF is a minimally invasive procedure and the fluid provides a quantitative source of biochemical indicators that reflects the response of the patient as well as the bacterial challenge.

Peri-implant sulcus fluid is the GCF-equivalent in the case a tooth is replaced with a dental implant, i.e. a fluid that flows from the site of the implant into the oral cavity.

GCF and peri-implant sulcus fluid is preferred if a site specific detection of the first and second substances is wanted, since distinct samples can be obtained from each examined tooth surface.

Saliva or mouth rinse samples may be used to obtain a detection or diagnosis that not is site specific.

As used here, the term "periodontal disease" and periodontitis shall be interpreted in its broadest sense to encompass such diseases as periodontitis, peri-implantatis (wherein the tissue supporting the implant is disintegrated), and other forms of periodontal disease as defined at the 1999 International workshop for classification of periodontal diseases and conditions.

Periodontal pathogenic bacteria, such as *Porphyromonas gingivalis, Bacteroides forsythus*, *Actinobacillus actinomycetemcomitans*, *Triponema denticola* and *Prevotella intermedia* can be present in the oral cavity and in the pocket between a tooth and the healthy gum without this leading to the progression of periodontal disease. The bacteria need certain growth conditions and presences of nutrients to become active. An active bacteria produces virulence products (such as the above mentioned toxins and enzymes) to aid its survival and nutrition.

Both *Porphyromonas gingivalis* and *Bacteroides forsythus* produce proteolytic enzymes known as trypsine-like serine proteases with a molecular weight of about 50 kDa. One of the proteases from *P*. *gingivalis* is named arg-gingipain, and one of the proteases from *B. forsythus* is a 48 kDa protease. *Actinobacillus actinomycetemcomitans* produce a 116 kDa leukotoxin which is a member of the repeats-in-toxin exoprotein family of pore-forming leukotoxins and is specifically cytotoxic to human polymorphonuclear leucocytes.

According to the invention, said first substance is a bacterial virulence product that is arg-gingipain from *Porphyromonas gingivalis.*

The presence of virulence products may trigger an inflammatory response and the immune system of the host, thus recruiting defence system cells, e.g. polymorphonuclear (PMN) leukocytes, to the site of infection.

The leukocytes cannot cope with the high amounts of bacteria and bacterial products at the infected site and enzymes from the leukocyte granulates are released into the periodontal pocket. These enzymes, originally aimed at killing the invading bacteria, are highly destructive to the surrounding tissues. Human neutrophil elastase has been shown to degrade many of the supportive tissues surrounding a tooth. Elastase is often found bound to its protease inhibitor (α-1 antitrypsin) in the periodontal pocket. However, the protease from *P*. *gingivalis* has been shown to degrade the protease inhibitors α-1 antitrypsin and α-2 macroglobulin in human serum, leaving elastase in its highly destructive form in the periodontal or peri-implant pocket.

As used herein, substances "originating from the immune or inflammatory system" refers to substances that originates from cells involved in the immune or inflammatory system. Such substances may be secreted from said cells, or may originate from lysis of such cells, for example to orchestrate the immune and inflammatory response or to remodell the tissue or killing the invading bacteria.

The second substance is a leukocyte product, a natural serine protease, human neutrophil elastase. Other substances comprise cytokine, such as an interleukin preferably chosen from among interleukin-1β, interleukin-6 and interleukin-8, or an inflammatory mediator, preferably tumour necrosis factor-α or possibly prostaglandin E₂.

Said second substance is human neutrophil elastase.

Other substances originating from the immune or inflammatory system of the patient suitable for detection according to the present invention comprise, but are not limited to, collagenases, aminotransferases, alkaline phosphatase, β-glucuronidase, dipeptidyl pedidase, neutrophil gelatinase-related lipocalin and matrix metalloproteinases.

Said first substance is a bacterial virulence product according to claim 1, and said second substance is human neutrophil elastase.

The co-existence of at least a first substance of bacterial origin and a second substance as defined above, human neutrophil elastase, should indicate both active bacteria and an active immune or inflammatory system, and this is indicative of periodontal disease. Thus it is advantageous to detect the co-existence of at least said first substances and said second substances.

In some instances, the test kit according to the present invention comprises additional detection assays for the detection of additional substances in said sample, and preferably such additional substances are selected among substances originating from bacteria as defined above.

The test kit according to the present invention comprises a first antibody exhibiting selective binding to said first substance, and comprises at a second antibody exhibiting a selective binding to said second substance.

As used herein, "antibody" refers to monoclonal antibodies, polyclonal antibodies, synthetically produced antibodies or antibody-equivalents and functional fragments thereof.

Antibodies of special interest for the present invention are antibodies that specifically binds to any of the above mentioned first or second substances. Antibodies raised against human neutrophil elastase are commercially available from MP Biomedicals. Antibodies against the leukotoxin from A. *actinomycetemcomitans* has been developed (Johansson et al. 2000) and antibodies against the protease from *P. gingivalis* has also been developed (Nakagawa et al. 2001).

As used herein, an "assay" refers to means for detecting the presence and/or determining the amount of one or more substance(s) in a sample. The result from an analysis on an assay is a detectable response, for example as a change in colour, fluorescence, absorbance and/or luminescence, a change in conductivity, a change in radioactivity etc.

Detection assays suitable for use with the test kit according to the present invention may be based on one out of several immunological methods. Such methods include, but are not limited to, immunochromatographic methods, immunometric methods, immunoagglutination methods, fluoroimmunological methods, immunoluminescence methods, turbidimetric immunolological methods, ELISA and nephelometric methods.

In a preferred embodiment of the test kit according to the present invention, said first and second detection assays provides immunochromatographic assays, preferably embodied as a so called "strip-test", implemented as lateral flow test. There are several different variants of immunochromatographic assays known to those skilled in the art. Each immunochromatographic assay preferably employs two antibodies specific for different epitopes of the substance to be detected.

Furthermore, more than one detection assay can be fitted onto one single strip-test, so that the presence of two or more substances specifically can be detected on one single strip.

Moreover, an immunochromatographic assay may be designed so that a predefined threshold amount of the sought substance is needed in the sample to yield a positive signal, as is known to those skilled in the art.

A method is provided for diagnosing periodontal diseases and/or predicting the risk for progress of said diseases, said method comprising analyzing a sample from the oral cavity of a patient for the presence of at least a first substance originating from bacteria and the presence of a second substance originating from the immune or inflammatory system of the patient.

In the method, sample from the oral cavity of a patient and the first and second substances are as defined above.

Also in some instances, the diagnostic method further comprises the step of detecting the presence of additional substances as defined above in said sample.

In preferred embodiments of the diagnosis method, said first method comprises using a first antibody exhibiting selective binding of said first substance and said second method comprises using a second antibody exhibiting selective binding of said second substance, wherein said antibodies are as defined above.

Most preferably at least one of said first and second methods comprises using an immunochromatographic method.

Other detection methods suitable for use with the methods may be based on one out of several immunological methods. Such methods include, but are not limited to, immunochromatographic methods, immunometric methods, immunoagglutination methods, fluoroimmunological methods, immunoluminescence methods, turbidimetric immunolological methods, ELISA and nephelometric methods.

### Preferred embodiments

Preferably a test kit according to the invention, as illustrated in figure 1, comprises two immunochromatographic detection assays 1, 2 arranged on a support 3 equipped with a sample reservoir 4 for receiving a sample. The two detection assays 1, 2 are arranged, directly or via a removably arranged separating means 5, in contact with the sample reservoir 4.

The sample reservoir 4 is preferably formed in the support material. The support 3 can be made of several different material, such as plastic, paper, carton or combinations thereof, such as paper laminated with plastic. The detection assays 1, 2 are fixed on the support 3 in such a manner that the sample receiving areas of each assay is, directly or via a removably arranged separating means 5, in contact with the sample reservoir 4. Said separating means 5 may be a removable foil covering the sample receiving areas on the assays, a dam separating the reservoir from the assays, etc.

The kit may further comprise additional buffers for dilution and adaptation of said sample for said detection assays and at least one sampling device for obtaining the sample to be analysed. The type of sampling device suitable for use may depend on the type of sample to be analysed. For example gingival crevicular fluid is a viscous fluid and can easily be collected by a small brush, a dental floss, a paper point or a disposable pipette. Other sampling devices for obtaining a saliva or mouth rinse sample are known to those skilled in the art. Preferably the buffer is kept separately from the sample reservoir in a buffer reservoir, such as a separate flask, an additional reservoir formed on the support or by providing the buffer in a puncturable bag placed in the sample reservoir.

After a sample is obtained, it is preferably mixed with buffer to obtain the pH, ionic strength and viscosity suitable for the detection assays. After mixing, the sample is transferred to the sample reservoir, and is thus, optionally by removing the separating means, brought in contact with a receiving area of the detection assays.

Through capillary flow, the sample is allowed to migrate along a cellulose strip to another area where small particles (for example colloidal gold or latex) coated with antibodies specific to one epitope of the protein (antigen) to be analysed by the detection assay is picked up by the liquid flow. Antigens present in the sample attaches to the particle bound antibodies and migrate further along the cellulose strip. Further down the path of the capillary flow, irreversibly bound to the strip, is another antibody (mono- or polyclonal) that recognise another epitope of the antigen. Particles that have caught an antigen attach to the area on the strip where the second antibody is bound, with the antigen sandwiched between the particle and strip bound antibodies respectively.

If enough particles get caught at the same area a visible line (test line) forms on the strip. Particles that have not caught an antigen continue down the strip and some get caught on a function control line. The particles are to small to be visible to the human eye one by one. Only if enough particles get caught in the same area a visible line forms.

The above process takes place essentially simultaneously in both strips, whereby one result for each of the analysed substances is obtained within a few minutes.

The above described preferred embodiment of the present invention and the following experiment is intended for illustrative purposes only, and shall not be interpreted as limiting the present invention. The scope of the present invention is defined by the appended claims.

### Experiments

### Material and methods

Samples for the following study were obtained from 16 volunteers who had been referred for periodontal treatment to the Department of Periodontology, Public dental service in Kristianstad, Sweden. The age of the patients ranged from 28 to 54 years with a mean of 40. All individuals had at least three sites with a probing depth greater than or equal to 6 mm, located at separate teeth. None of the patients were medically compromised or had periodontal and/or antibiotic treatment during the preceding 6 months.

Sampling and clinical examination were preformed in duplicate, 1 week apart. The samples for enzyme analysis were collected before microbial sampling and clinical examination. The first sampling and examination was performed before any treatment was given (baseline, determination 1). The second sampling and examination occurred 6 months after completion of the first treatment (determination 2), and the third, another 6 months after the second treatment was finished (determination 3).

After baseline examination all patients received oral hygiene instructions and supra- and subgingival debridement throughout the entire dentition. At the second treatment session periodontal surgery or rescaling under anaesthesia was preformed at all sites where P. *gingivalis* constituted ≥ 0,5% or *P*. *Intermedia* ≥ 5 % of the total anaerobic viable count, or A. *actinomycetemcomitans* was present in the subgingival samples. All other sites selected for the study received supragingival polish.

From each patient, samples were collected from 3 to 10 sites with initial probing depths ≥ 6 mm. The sampling area was dried and isolated with cotton rolls and, supragingivally, plaque was carefully removed with sterile curets and cotton pledglets. For the enzymes assays, three medium paper points (Johnson and Johnson, Windsor, NJ) were inserted consecutively approximately 1 mm into the peridontal sulcus and left for 15 seconds. The wet part of each paper point was cut of with a sterile pair of scissors and pooled in a minisorb tube (Nunc, Rosklide, Denmark) containing 100 µl 0,85% NaCl. The samples were frozen immediately at -20°C and within 6 hours at -80°C, and stored until assayed. For the microbial analysis 3 paper points were inserted consecutively into the periodontal pocket until resistance was met and left in place for 15 secondes. The points were pooled into a vial containing 10 glass beads, 3 mm in diameter, and 3,3 ml of VMGA III transport medium, aerobically prepared and stored. The samples were processed within 24 hours. Bacteria were grown on enriched Brucella agar plates and identified by appropriate methods.

For the study of appropriate cut-off values for a diagnostic test we studied elastase from human neutrophils and arg-gingipain from *P*. *gingivalis* using selective substrates.

The first set of the duplicate samples was used for the enzyme assays. All samples were thawed on ice and centrifuged for 3 minutes at 13,000 × g.

The enzyme substrate for determining arg-gingipain from *P*. *gingivalis* was N-benzoyl-L-arginine-p-nitroanilie (BAPNA) with a final concentration of 1 mM in the assay buffer containing 5 % DMSO. The assay buffer was 0.1 M Tris-HCl containing 5 mM CaCl, pH 7,5, with 50 mM glycyl-glycine (as reported earlier by the inventors in patent US 5,981,164 gly-gly stimulates BAPNA selectively in the presence of arg-gingipain) and 5 mM L-cysteine. Ten µL of the sample was preincubated for 15 minutes with 140 µL of the assay buffer in the well of a 96-well microtiter plate, precoated with bovine serum albumin, before 50 µL of the substrate was added. The plate was incubated at 37°C in a humid chamber, and the release of pNA was followed spectrophotometrically by OD₄₀₅ readings, using a microtiter plate reader, every few hours from 12 to 36 hours. One unit of activity was equal to the amount of enzyme which cleaved 1 nmol of the substrate during one hour of incubation.

The elastase assay was preformed by adding 5 µl of CGF to a well of a 96-well microtiter plate containing 145 µl of an assay buffer (0,1 M Tris, 0,5 M NaCl at pH 7,5). After 15 minutes the reaction was stared by adding 50 µl of a 2 mM solution of Methoxysuccinly-Ala-Ala-Pro-Valine-pNA in assay buffer containing 20% DMSO. The plate was incubated at 37°C in a humidified chamber. The enzymatic reaction releasing the pNA was read at OD₄₀₅ every few hours from 12 to 26 hours using a microtiter plate reader (Molecular Devices) and compared to a standard of diluted purified enzyme. The recorded readings were plotted against time and enzyme activity was calculated as DA₄₀₅/60 minutes from the linear part of the plot. The amount of elastase was presented in ng per site.

Patients with growth of *P*. *gingivalis* or presence of gly-gly stimulated BAPNA activity but with no growth of *A. actinomycetemcomitans* where included in the cut-off study. This criterion yielded 35 sites from 8 different patients. The determination one year after initial treatment was used in this limited study. Attachment loss as measured from the bottom of the periodontal pocket to the root-cement margin with a pressure balanced probe was used as a measurement of further progression of periodontal decease. Elastase and arg-gingipain were analysed for their ability to predict futher attatchment loss. Individually the elastase cut-off level were set to 20 ng per site and for arg-gingipain to 0,27 units per site.

### Results

| Elastase | Attachment loss | Attachment gain or zero |
|---|---|---|
| Positive test (Elastase >20 ng) | 3 | 2 |
| Negative test (Elastase ≤20 ng) | 3 | 27 |

Elastase as a predictor of further attachment loss yielded a sensitivity of 50% and a specificity of 93%. A p-value of 0.0264* was calculated using Fisher's exact test.

| Arg-gingipain | Attachment loss | Attachment gain or zero |
|---|---|---|
| Positive test (arg-gingipain >0,27 U) | 6 | 13 |
| Negative test (arg-gingipain ≤0,27 U) | 0 | 16 |

Arg-gingipain as a predictor of further attachment loss yielded a sensitivity of 100% and a specificity of 55%. A p-value of 0.0216* was calculated using Fisher's exact test.

For elastase and arg-gingipain alone either the senistivity or the specificity had to be sacificed. This promted us to investigate the combination of the two enzymes. We investigated new cut-off levels for the two enzymes to find that the detection limit for elastase should be 2 ng per site and 0,30 units for arg-ginipain.

| Combination | Attachment loss | Attachment gain or zero |
|---|---|---|
| Positive test (Elastase >2 ng and arg-gingipian >0,30 U) | 5 | 3 |
| Negative test (Elastase ≤2 ng or arg-gingipain ≤0,30 U) | 1 | 26 |

The combination of elastase and arg-gingipain as a predictor of further attachment loss yielded a sensitivity of 83% and a specificity of 90%. A p-value of less than 0,001*** was calculated using Fisher's exact test. This limited data shows that the combination of elastase and arg-gingipain as a marker for periodontal diesease yields a statistically more significant test than either of the enzymes alone.

### References

Armitage G.C. (2003) Position paper: Diagnosis of Periodontal Diseases. Journal of Periodontology 74, 1237-1247.
Chapple I.L.C (1997) Periodontal disease diagnosis: current status and future developments. Journal of Dentistry 25, 3-15.
Eley B.M. and Cox S.W. (1996) Correlation Between Gingivain/Gingipain and Bacterial Dipeptidyl Peptidase Activity in Gingival Crevicular Fluid and Periodontal Attachment Loss in Chronic Periodontitis Patients. A 2-Year Longitudinal Study. Journal of Periodontology 67, 703-716.
Eley B.M. and Cox S.W. (2003) Proteolytic and hydrolytic enzymes from putative periodontal pathogens: characterization, molecular genetics, effects on host defences and tissues and detection in gingival crevice fluid. Periodontology 2000, 31, 105-124.
Jin L.J., Söder P-Ö. , Leung W.K., Corbet E.F., Samaranayake L.P., Söder B. and Davies W.I.R. (1999) Granulocyte elastase activity and PGE2 levels in gingival crevicular fluid in relation to the presence of subgingival periodontopathogens in subjects with untreated adult periodontitis. Journal of Clinical Periodontology 26, 531-540.
Johansson A., Sandström G., Claesson R., Hänström L., Kalfas S. (2000) Anaerobic neutrophil-dependent killing of Actinobacillus actinomycetemcomitans in relation to the bacterial leukotoxicity. European Journal of Oral Sciences 108, 136-149.
Lamster I.B., Smith Q.T., Celenti R.S., Singer R.E. and Grbic J.T. (1994) Development of a risk profile for periodontal disease: microbial and host response factors. Journal of Periodontology 65 (5 Suppl), 511-20.
Nakagawa, T., Sims, T., Fan, Q., Potempa, J., Travis, J., Houston, L. and Page, R.C. (2001) Functional characteristics of antibodies induced by Arg-gingipain (HRgpA) and Lys-gingipain (Kgp) from Porphyromonas gingivalis. Oral Microbiology and Immunology 16 (4), 202-211.
Nisengard R.J., Mikulski L., McDuffie D. and Bronson P. (1992) Development of a rapid latex agglutination test for periodontal pathogens. Journal of Periodontology 63 (7), 611-617.
Oringer R.J. (2002) Modulation of the Host Response in Periodontal Therapy. Journal of Periodontology 73, 460-470.
Socransky S.S., Smith C., Martin L., Paster B.J., Dewhirst F.E. and Levin A.E. (1994) "Checkerboard" DNA-DNA hybridization. Biotechniques 17 (4), 788-92.

## Claims

1. A test kit for detecting periodontal disease in a patient by analysing a sample from the oral cavity of the patient, wherein said kit at least comprises:
a first detection assay for detecting a first substance originating from bacteria, said first detection assay comprising:
i. a first antibody exhibiting selective binding to said first substance, and
ii. means for generating a detectable response,
said first substance being arg-gingipain from Por*phyromonas gingivalis,* and
a second detection assay for detecting a second substance originating from the immune or inflammatory system of the patient, said second detection assay comprising:
i. a second antibody exhibiting selective binding to said second substance, and
ii. means for generating a detectable response,
said second substance being a human neutrophil elastase.

2. A test kit according to claim 1, wherein each of said first and second detection assays provides an immunochromatographic assay.

3. A test kit according to any of the preceding claims, further comprising a support provided with a sample reservoir for receiving said sample, wherein said first and second detection assays are arranged on said support in contact with said sample reservoir, directly or via a removably arranged separating means which separates said sample reservoir from said detection assays.

4. A test kit according to any of the preceding claims, further comprising additional buffers for dilution and adaptation of said sample for said detection assays.

5. A test kit according to claim 4, further comprising a buffer reservoir separate from said sample reservoir.

6. A test kit according to any of the preceding claims, further comprising at least one sampling device for obtaining said sample.

## Patentansprüche

1. Testkit zum Nachweis einer peridontalen Erkrankung bei einem Patienten durch Analyse einer Probe aus der Mundhöhle des Patienten, wobei das Kit mindestens Folgendes umfasst:
ein erstes Nachweis-Assay zum Nachweis einer ersten Substanz, die aus Bakterien stammt, wobei das erste Nachweis-Assay Folgendes umfasst:
i. einen ersten Antikörper, der selektive Bindung an die erste Substanz aufweist, und
ii. Mittel zum Erzeugen einer nachweisbaren Reaktion,
wobei die erste Substanz Arg-Gingipain aus *Porphyromonas gingivalis* ist, und
ein zweites Nachweis-Assay zum Nachweis einer zweiten Substanz, die aus dem Immun- oder Entzündungssystem des Patienten stammt, wobei das zweite Nachweis-Assay Folgendes umfasst:
i. einen zweiten Antikörper, der selektive Bindung an die zweite Substanz aufweist, und
ii. Mittel zum Erzeugen einer nachweisbaren Reaktion,
wobei die zweite Substanz eine humane Neutrophilenelastase ist.

2. Testkit gemäß Anspruch 1, wobei jedes des ersten und zweiten Nachweis-Assays ein immunchromatographisches Assay bereitstellt.

3. Testkit gemäß eines der vorhergehenden Ansprüche, ferner umfassend eine Haltevorrichtung versehen mit einem Probenreservoir zum Aufnehmen der Probe, wobei das erste und zweite Nachweis-Assay auf der Haltevorrichtung in Kontakt mit dem Probenreservoir angeordnet sind, direkt oder über ein entfernbar angeordnetes Trennmittel, welches das Probenreservoir von den Nachweis-Assays trennt.

4. Testkit gemäß eines der vorhergehenden Ansprüche, ferner umfassend zusätzliche Puffer zum Verdünnen und Anpassen der Probe für die Nachweis-Assays.

5. Testkit gemäß Anspruch 4, ferner umfassend ein Pufferreservoir getrennt von dem Probenreservoir.

6. Testkit gemäß eines der vorhergehenden Ansprüche, ferner umfassend mindestens ein Probenahmegerät zum Erhalten der Probe.

## Revendications

1. Trousse de dosage pour la détection d'une maladie périodontique chez un patient par l'analyse d'un échantillon provenant de la cavité buccale du patient, ladite trousse comprenant au moins :
un premier dosage de détection pour détecter une première substance provenant de bactéries, ledit premier dosage de détection comprenant :
i. un premier anticorps exhibant une liaison sélective vis-à-vis de ladite première substance, et
ii. un moyen pour générer une réponse détectable,
ladite première substance étant l'arg-gingipaïne issue de *Porphyromonas gingivalis,* et
un second dosage de détection pour détecter une seconde substance provenant du système immunitaire ou inflammatoire du patient, ledit second dosage de détection comprenant :
i. un second anticorps exhibant une liaison sélective vis-à-vis de ladite seconde substance, et
ii. un moyen pour générer une réponse détectable,
ladite seconde substance étant une élastase neutrophile humaine.

2. Trousse de dosage selon la revendication 1, dans laquelle chacun desdits premier et second dosages de détection offre un dosage immunochromatographique.

3. Trousse de dosage selon l'une quelconque des revendications précédentes, comprenant en outre un support doté d'un réservoir d'échantillon pour la réception dudit échantillon, dans laquelle lesdits premier et second dosages de détection sont arrangés sur ledit support en contact avec ledit réservoir d'échantillon, directement ou par le biais d'un moyen de séparation arrangé de manière amovible qui sépare ledit réservoir d'échantillon desdits dosages de détection.

4. Trousse de dosage selon l'une quelconque des revendications précédentes, comprenant en outre des tampons supplémentaires pour la dilution et l'adaptation dudit échantillon pour lesdits dosages de détection.

5. Trousse de dosage selon la revendication 4, comprenant en outre un réservoir de tampon séparé dudit réservoir d'échantillon.

6. Trousse de dosage selon l'une quelconque des revendications précédentes, comprenant en outre au moins un dispositif d'échantillonnage pour l'obtention dudit échantillon.
